# EUROPEAN PATENT APPLICATION

(11) **EP 1 013 776 A1**
(43) Date of publication of application: **28.06.2000**
(21) Application number: 98204421.6
(22) Date of filing: 22.12.1998
(51) Int. Cl.: C12Q 1/68, C12Q 1/70

(54) **A sensitive assay for the detection or quantitation of human cytomegalovirus nucleic acid**

(71) Applicant: Universiteit van Amsterdam, 1012 WX Amsterdam (NL)
(72) Inventor: The designation of the inventor has not yet been filed
(74) Representative: Ottevangers, Sietse Ulbe

(57) **Abstract**

Tools and methods for the detection or quantification of human cytomegalovirus (CMV) in a sample by means of amplification of human CMV nucleic acid. The primers used for the amplification of CMV nucleic acid are chosen from regions in CMV that are conserved in nine different isolates of CMV. Presented are DNA extraction methods, amplification primers and a specifically designed internal control DNA especially suited for the detection or quantification of a wide variety of different strains of human CMV. The methods and tools are especially suited for the detection of low amounts of CMV nucleic acid in serum and plasma but may also be used for the detection in other samples such as, but not limited to, whole blood or cerebrospinal fluid.

## Description

### FIELD OF THE INVENTION

The invention lies in the field of nucleic acid amplification. More specifically the invention lies in the field of nucleic acid amplification for the detection of a human cytomegalovirus nucleic acid in a sample.

### BACKGROUND TO THE INVENTION

Infection with cytomegalovirus (CMV) is an important cause of morbidity and mortality in immunocompromised individuals, like transplant recipients, AIDS patients and new-borns. For monitoring the patients at risk for developing CMV disease and for the monitoring of treatment, rapid, specific, and sensitive tests are needed. One such test is the polymerase chain reaction (PCR, 31) which can detect CMV DNA present in the blood compartment as cell associated virus in the white blood cells, and as free virus in serum and plasma.

Since the first detection of CMV in serum and plasma (22, 35, 6), many reports have appeared on the qualitative (diagnostic) detection of CMV DNA in plasma or serum. In bone marrow transplant recipients (22, 38, 3, 27, 20), renal transplant recipients (6, 38, 15, 10), and liver transplant recipients (28, 32, 15, 29), the presence of CMV DNA in plasma or serum has been shown to be an early marker for CMV infection and CMV disease. In new-borns with congenital CMV infection (25) and in children with AIDS (26) CMV can be readily detected in serum. In HIV seropositive patients, the presence of CMV DNA in plasma or serum has been shown to precede CMV disease by median 46 days (13) to 3 months (19). Although the negative predictive power for disease in HIV seropositive patients is high, the positive predictive power is still a matter of debate (13, 24). In general, levels of CMV DNA found in plasma or serum were significantly lower than those found in white blood cells (14, 42), therefore very sensitive assays are needed for CMV detection in such specimens.

Most of the diagnostic procedures for the detection of CMV in serum and plasma cited above are not readily implemented in the routine setting of a clinical virology laboratory since these involve gel electrophoresis, southern- or slot-blotting followed by hybridisation with (radioactively) labelled probes in order to reach sufficient sensitivity. In addition, nested PCR was frequently used to reach the same goal. The use of nested PCR precludes the use of N-Uracil-glycosylase (UNG system, Perkin Elmer) to avoid false positives due to amplimer carry-over, a system which was only used by two groups of investigators (16, 27). Another problem in diagnostic detection is the preparation of CMV DNA from serum and plasma. Although simple pretreatments of these specimens (e.g. proteinase digestion followed by heat treatment, or treatment with alkali) usually will give positive results at high viral loads, low CMV loads will remain undetected (3, 22). In all studies where CMV DNA was purified from the specimen before PCR, a control monitoring for the DNA extraction efficiency was not included. Likewise, controls monitoring for the presence of PCR inhibitors were usually absent. This has led to the situation that in most of the diagnostic assays described above, the sensitivity of the assay was unknown, which made interpretation of the diagnostic data difficult.

Quantitation of CMV in serum or plasma is important for the detection of patients at highest risk for disease, for monitoring the efficacy of therapy, the development of resistance and for prognosis. To this end, several procedures for the quantitative assessment of CMV in serum and plasma have been developed. Some of these procedures are based on limiting dilution followed by nested PCR or detection with radioactively labelled probes (35, 37). Others are based on co-amplification of internal control DNA (competitive PCR) which shares the primer sequences with the target of interest but generates an altered PCR product (18, 7, 42, 34, 16, 40, 33). Such internal DNA corrects for variations in PCR efficiency due to e.g. inhibitory substances and well-to-well variation of the thermocycler (12, 9). With the present invention a method is made available that enables the user to obtain absolute quantitative information by competitive PCR. The principle of this aspect of the invention is that target and internal control DNA are amplified with equal efficiencies using at least one identical primer pair. Under these conditions the initial ratio of target to competitor remains constant throughout amplification (including plateau phase) and this ratio can be used to calculate the viral load, provided that the amplification products are accurately measured (9). Measurement of amplification products has been done by HPLC (7) and by hybridisation with ³²P-labeled probes followed by PAGE (34, 33) which are procedures that are not readily implemented in a routine clinical virology laboratory. Amplification products have also been measured after slot blotting and hybridisation with digoxigenin labelled probes followed by enzymatic conversion of a substrate into a coloured reaction product which is subsequently measured by light absorbance (18). Recently, a plate assay has been described (30, 42, 16, 40) in which amplification products, containing a 5' biotinylated strand, were hybridised to the corresponding specific detector probe bound to wells. The amount of hybrids was subsequently quantified spectrophotometrically after enzymatic conversion of substrate. Since the ratios of CMV signals over IC signals are not a direct reflection of the amount of CMV, an external reference curve has to be prepared for each assay.

The present invention comprises highly sensitive diagnostic assays (D-PCR) for the detection of CMV. In one embodiment of the invention, internal control (IC) DNA, mimicking the CMV target, is included in the DNA extraction and all subsequent steps of the procedure, monitoring for both DNA extraction efficiency and for the presence of PCR inhibitors, thus excluding false negative reactions. In one embodiment of the invention the UNG system is used to prevent false-positives due to amplimer carry-over. High sensitivity and specificity is achieved by solution hybridisation with, for example, non-radioactive, {Tris (2,2'-bipyridine) ruthenium (II) chelate) (TBR) -labelled probes which discriminate between CMV and IC amplimers. The amount of hybrids can subsequently be determined by electrochemiluminescence (ECL) in the Perkin Elmer System 5000. The present invention further provides a high performance quantitative assay (Q-PCR) for the detection of human CMV in a sample. The Q-PCR is similar to the D-PCR except for the presence of an additional fixed amount (in one embodiment of the invention, 35 molecules) of IC DNA during PCR. The viral load (expressed as copies CMV/ml) is calculated by a straightforward algorithm from the ratio of CMV over IC signals obtained after solution hybridisation with TBR labelled probes and ECL measurement. The Q-PCR will reliably detect 4-fold differences in viral load in the range of 100 to 150,000 copies CMV/ml. The Q-PCR is equally applicable for serum and plasma regardless of the anticoagulant.

### SUMMARY OF THE INVENTION

The present invention provides tools and provides methods for the detection or quantification of human cytomegalovirus nucleic acid in a sample. The methods of the invention are based on amplification of human CMV nucleic acid. The primers used for the amplification of CMV nucleic acid are chosen from regions in CMV that are conserved in nine different isolates of CMV. The present invention teaches DNA extraction methods, choice of amplification primers and a specific design and use of internal control DNA especially suited for the detection or quantification of a wide variety of different strains of human CMV. The methods and tools of the invention are especially suited for the detection of low amounts of CMV nucleic acid in serum and plasma but may also be used for the detection in other samples such as, but not limited to, whole blood or cerebrospinal fluid.

### BRIEF DESCRIPTION OF THE DRAWINGS

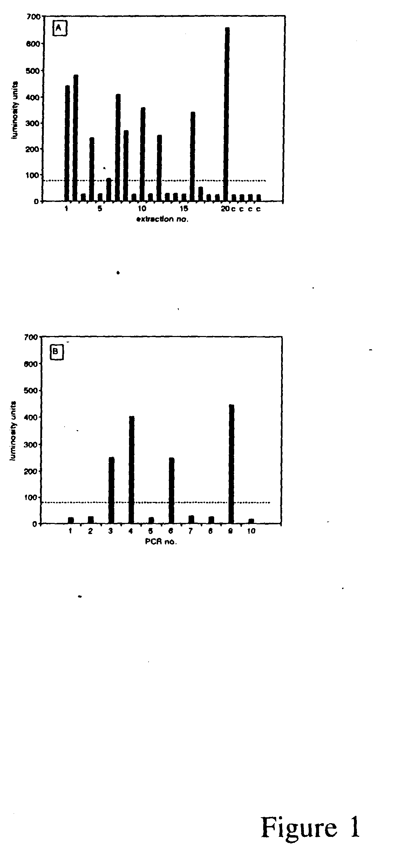

Figure 1. Recovery of IC DNA from plasma at the single molecule level.
   A. Reference plasma was supplemented with IC DNA to a concentration of 4 molecules per 200 µl plasma. DNA was extracted (in 20-fold) from 200 µl samples and one quarter of the extracted DNA was used in the PCR reaction (lanes 1-20). DNA was also extracted (in 4-fold) from reference plasma only, which served as negative control (c). The amount of amplimers was determined by ECL and expressed in luminosity units. Cut off level (80 luminosity units) is indicated by a dotted line.
   B. In the same experiment IC DNA was used *directly* in 10 separate PCR reactions at an input expected to contain a mean of 1 molecule of IC DNA per PCR reaction (lanes 1-10).

   The amount of amplimers was determined by ECL and expressed in luminosity units. Cut off level (80 luminosity units) is indicated by a dotted line.

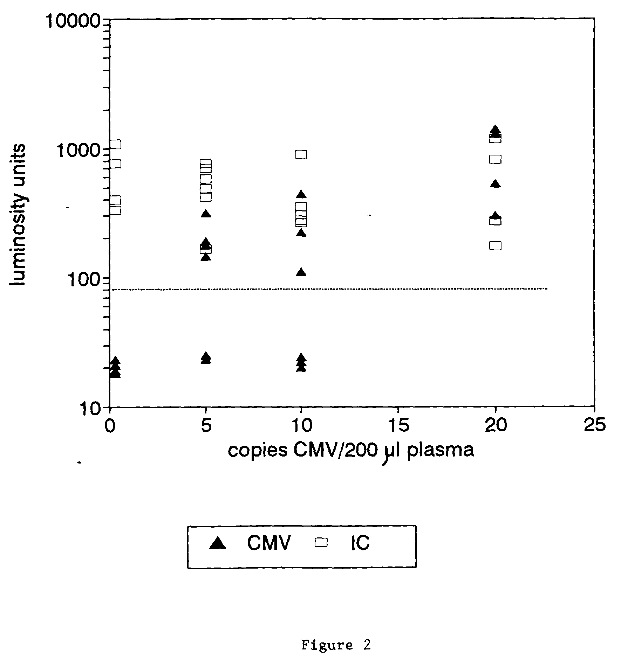

Figure 2. Lower limit of detection of CMV DNA in plasma. DNA was purified from 200 µl reference plasma samples containing 20, 10, 5, or 0 CMV virus particles; 35 molecules of IC DNA were co-extracted. Extractions were done in four-fold for the specimens containing 20, and 0 particles, and in six-fold for specimens containing 10 and 5 particles. One-fifth of extracted DNA was subjected to a 35 cycle PCR and the amount of CMV-specific amplimers (filled triangles) and IC-specific amplimers (open squares) was determined by ECL after hybridisation with CMV-specific and IC-specific TBR-labelled probes (expressed in luminosity units). The cut-off level (80 luminosity units) is indicated by a dotted line.

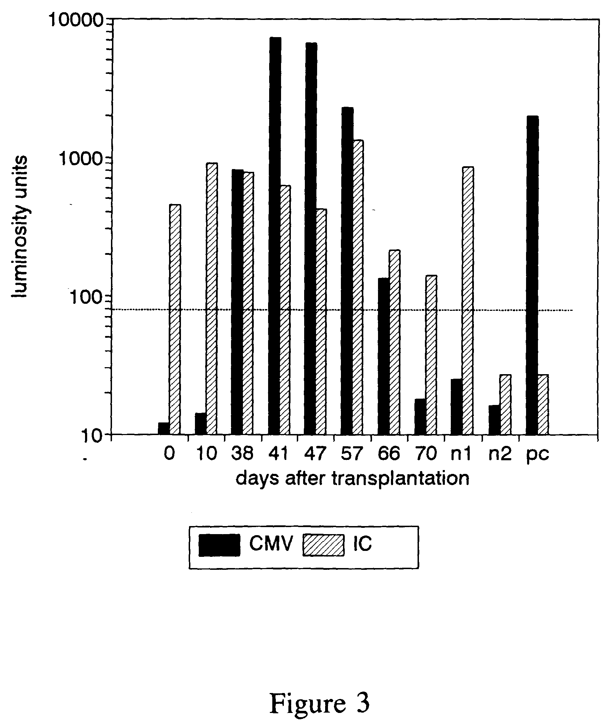

Figure 3. Diagnostic PCR. Sequential serum specimens from a renal transplant patient were subjected to the D-PCR. Two negative controls (n1 = human DNA + 35 molecules IC DNA, and n2 = human DNA only), and one positive control (pc; a plasma specimen containing 400 copies CMV/ml), were included. Amplimers were detected by hybridisation with IC-specific probe and CMV-specific probe, and the amount of amplimers was expressed in luminosity units. Filled bars, CMV-specific probe; hatched bars, IC-specific probe. The cut-off level is indicated by a dotted line.

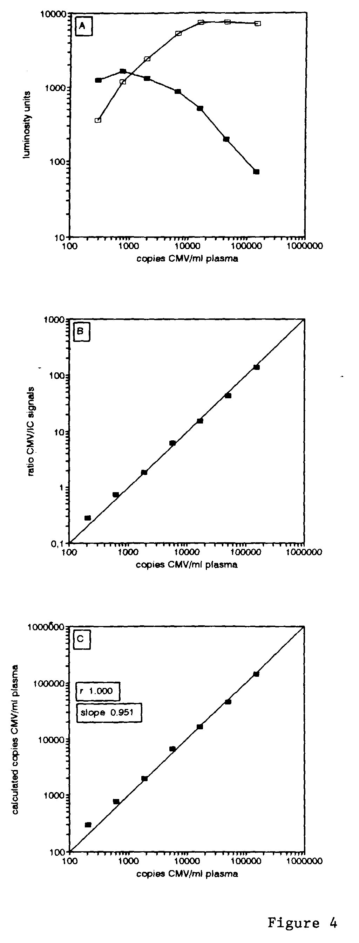

Figure 4. Quantitative PCR. Reference plasma was supplemented with CMV particles to a concentration of 150.000 copies CMV/ml, and serial three-fold dilutions were made in the same plasma to reach concentrations down to 206 copies CMV/ml. Q-PCR was done for this dilution series as described in Materials. A), ECL signals (luminosity units) obtained by Q-PCR after hybridisation with CMV-specific probe (open squares) and IC-specific probe (filled squares). B), the corresponding values of the CMV/IC ratios calculated from ECL signals after background subtraction. C), CMV loads were calculated directly from the CMV/IC ratios by the algorithm described in Materials and expressed in copies CMV/ml plasma. Negative controls were included (reference plasma only); calculated loads for these controls varied between 0 and -2 copies/ml. Correlation coefficient (r) and slope were obtained by least squares linear regression analysis.

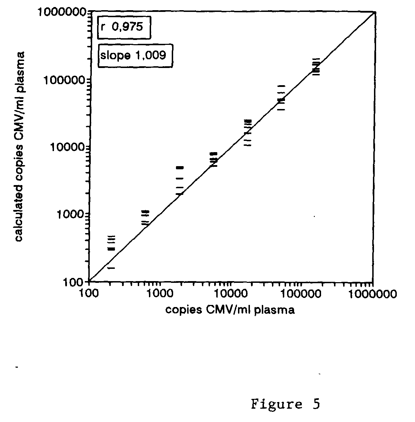

Figure 5. Inter-assay variation. The inter-assay variation of the Q-PCR was determined from 7 separate Q-PCR assays (performed on different days over a 3 month period) for the plasma dilution series described in the legend to Figure 4. Correlation coefficient (r) and slope were calculated by least squares linear regression analysis for all data points (n=54).

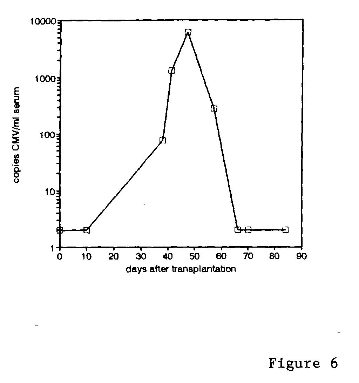

Figure 6. Quantitation of CMV load by Q-PCR. The sequential serum specimens from the renal transplant patient described in Figure 3 were subjected to Q-PCR and the CMV load was expressed as copies CMV/ml serum. Specimens found negative were arbitrarily plotted at 2 copies CMV/ml for graphical representation.

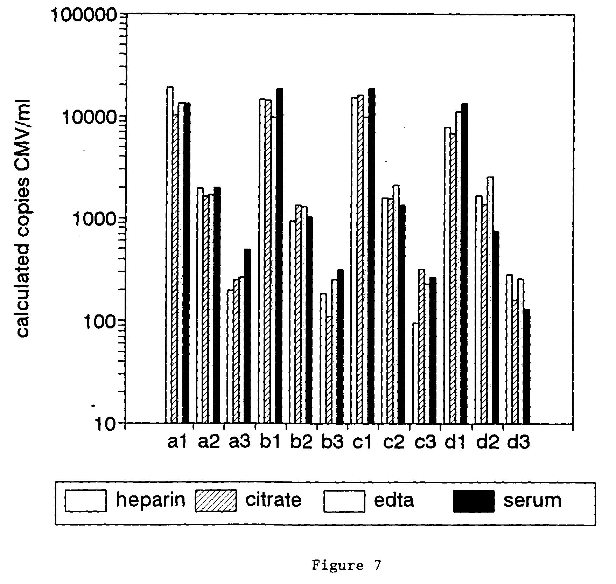

Figure 7. Serum and plasma specimens are equivalent substrates for Q-PCR. Serum, heparin plasma, EDTA plasma, and citrate plasma obtained from each of 4 (a, b, c and d) healthy volunteers (subject a, CMV-seronegative; subjects b-d, CMV-seropositive) were seeded with CMV to 15,000 copies CMV/ml (a1, b1, c1, d1), 1,000 copies CMV/ml (a2, b2, c2, d2) and 185 copies CMV/ml (a3, b3, c3, d3) and Q-PCR was done for each of these specimens. Calculated loads are given in copies CMV/ml. Open bars, heparin plasma; hatched bars, citrate plasma; stippled bars, EDTA plasma; filled bars, serum.

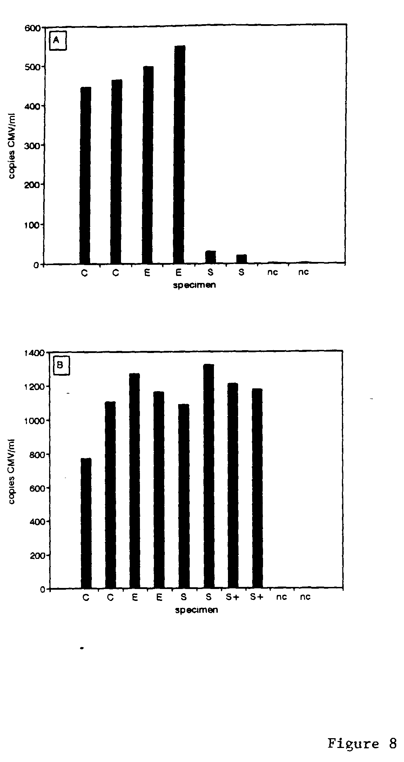

Figure 8. CMV loads in patient serum may be significantly lower than those observed in plasma. A. Citrate plasma (C), EDTA plasma (E), and serum (S), obtained in parallel from the same patient (subject A in Table 2), was subjected to Q-PCR (in duplicate) and the calculated CMV loads in copies CMV/ml are given. B. Citrate plasma (C), EDTA plasma (E), and serum (S), obtained from a CMV DNA-negative healthy volunteer and the serum from the patient (S+) were seeded to 750 copies CMV/ml and subjected to Q-PCR. The calculated CMV loads are given in copies CMV/ml; nc, negative controls (reference plasma only).

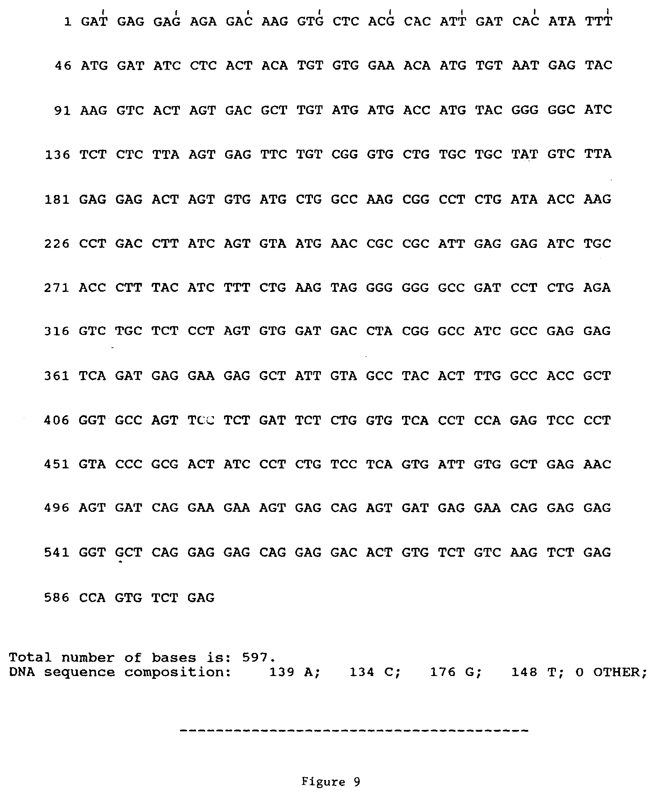

Figure 9. Nucleotide sequence of the IC-DNA described in the Materials and Methods section.
   Table 1. Inter assay and intra assay variation. Human reference plasma was supplemented with CMV to 150,000 copies/ml and serial threefold dilutions were made in the same plasma. Q-PCR was done for these specimens to determine inter assay (n=7) and intra assay (n=8) variation. Calculated mean values and coefficients of variation are given.
   Table 2. Comparison of CMV DNA loads in serum and plasma in patient specimens. CMV DNA loads were determined by Q-PCR in paired serum and plasma specimens from 5 renal transplant patients (RTX), one AIDS patient and one ABMT (allogeneic bone marrow transplantation) patient; days after transplantation are given where applicable. Data for subjects A, B, C and E were obtained from duplicate Q-PCR assays. NA, not available.

### DETAILED DESCIPTION OF THE INVENTION

The methods and tools of the invention are especially suited for the detection or quantification of a wide variety of human cytomegalovirus in clinical samples. The primers for the amplification are chosen from a region perfectly conserved in nine different isolates of human CMV. The methods and tools of the invention also comprise special methods and buffers for the extraction of nucleic acid from clinical samples. We have previously reported on a reliable procedure for the purification of nucleic acids from clinical specimens (Silica-GuSCN procedure, 5). This procedure has been used for the extraction of CMV DNA from clinical specimens like white blood cells (41, 17), urine (2), aqueous humour (17), serum (24), and plasma (18) and has been acknowledged for its potency to remove substances inhibitory for PCR. At present no published information is available concerning DNA extraction efficiency at very low DNA-concentrations. In the present invention we have adapted the Silica-GuSCN procedure by supplementing the original lysis buffer with alpha casein. The data show that DNA recovery from plasma was about 100%. We demonstrated that this buffer was efficient and reproducible in the isolation of DNA from samples with a very low concentration of DNA. Even at extremely low DNA inputs for extraction (4 DNA molecules of IC DNA present in 200 µl of plasma) yields were near 100% (Figure 1). Similar high yields were also obtained using a linearised 10 kb plasmid (pES, 4) containing the CMV immediate-early region rather than IC DNA (data not shown).

The methods of the invention comprise a highly sensitive diagnostic (D-PCR) and quantitative assay (Q-PCR) for the detection of CMV in samples such as in plasma and serum. The methods of the invention were validated using test runs comprising a total of 20 clinical specimens and four controls per run (either by D-PCR or Q-PCR). The specimens and the controls were evaluated within an 8 h working day. Costs per specimen for materials were about 10 US dollars, hands-on time was about 5 hours. The methods of the invention are suited to test, within a working day, at least 24 samples per run.

The Q-PCR method of the invention was in addition to the procedure mentioned above, also validated by determining the copy number of CMV immediate early genes which are stably integrated into the chromosomal DNA of Rat-9G cells (4). Copy numbers previously determined by southern blot analysis (4) and values determined by Q-PCR (about 15 copies IE gene/cell) were in good accordance suggesting that high molecular weight DNA was also isolated with very high efficiency (data not shown). The high DNA extraction efficiency in combination with the sensitive PCR methods of the invention, resulted in a highly sensitive diagnostic assay which is capable of detecting CMV in 4 out of 6 reactions at a viral load as low as 25 copies CMV/ml plasma.

The function of the internal control DNA is two-fold. The first reason to add the internal control DNA is to provide a reference molecule to control the DNA extraction procedure. Because the IC DNA is specifically added in the first step of DNA extraction, together with the sample, it is known that the IC DNA is present and thus should be detected with the methods of the invention. A failure to do so in a sample indicates that the procedure for this sample resulted in an insufficiently sensitive detection level. The second function of the internal control DNA is to provide a known amount of reference DNA molecules for amplification to which a signal from amplified product of CMV nucleic acid can be related and quantified. In the D-PCR procedure of the invention the IC-DNA is added only to the sample, preferably in the amount of 35 molecules. In the Q-PCR procedure of the invention the important aspect is to have a known amount of IC DNA present during the amplification step. For this reason a known amount, preferably 35 molecules, of IC DNA is added to the PCR-reaction mix. For most purposes, the Q-PCR procedure is carried out on samples that gave rise to a detectable signal, i.e. had detectable amplified product of human CMV nucleic acid, in the D-PCR procedure. Thus a certain amount of IC-DNA is already present in the reaction mix before the Q-PCR method is carried out. In case of the preferred embodiment at this stage an extra amount of 35 molecules of IC DNA is added to the reaction mix. However, in other embodiments of the invention the amount of IC DNA added to the sample in the D-PCR is sufficiently high for quantification purposes. In this case no extra IC DNA needs to be added for the Q-PCR procedure. In essence the results, i.e. the amplified products, of the D-PCR procedure can be used directly for quantification purposes.

However, in the preferred embodiment of the invention, 35 molecules of IC DNA are added to the sample for the D-PCR procedure and an extra 35 IC DNA molecules are added to the reaction mix for the Q-PCR procedure. This additional IC DNA serves to dampen variation due to Poisson distribution and to extend the dynamic range of the Q-PCR. Following PCR, the amounts of CMV-specific and IC-specific amplimers may be determined after solution hybridisation with TBR-labelled probes followed by ECL in the QPCR System 5000 (Perkin Elmer). CMV loads are calculated from the ratio of CMV/IC ECL signals by the most straightforward algorithm possible which assumes ideal circumstances for each of the steps of the Q-PCR. Under these conditions the ratio of CMV/IC ECL signals is a direct reflection of the amount of CMV present in the clinical specimen. The data presented here show that the expected and calculated CMV loads are in good accordance. Mean interassay, intra-assay and interspecimen variation of the Q-PCR are about 25% suggesting that the Q-PCR will reliably detect 4-fold differences in viral load in serum and plasma specimens in the range of 200 to 150,000 copies CMV/ml. Additional experiments, not shown here, have demonstrated that loads down to about 100 copies/ml are reliably quantitated. The contribution of the variation in DNA yield to the total observed variation in the Q-PCR is relatively small, about 7% of a total variation of about 23% for serum specimens obtained from different patients. When the contribution of the variation in DNA yield to the total observed variation in the Q-PCR is determined using a single serum specimen only approximately 2% of a total variation of approximately 18% is ascribable to variation in extraction efficiency (not shown).

Since in the preferred embodiment of the D-PCR the procedure is internally controlled by a known (but very low, only 35 molecules are added to the sample) number of IC DNA molecules, quantitative information can also be obtained from D-PCR data by an algorithm (copies CMV/ml = R x 175) which is based on the same criteria as described in the Methods section for Q-PCR. Quantitative data can be obtained by either D-PCR or Q-PCR which results in similar data in the lower range (100-50,000 copies CMV/ml). However, the observed variation in the amount of virus nucleic acid detected in the preferred embodiment of the D-PCR method, make this method of the invention better suited for the analysis of the presence or absence of CMV nucleic acid in a sample. When high amounts of CMV nucleic acid needs to be quantified the Q-PCR is preferred.

The Q-PCR and the D-PCR method of the invention are suited for the analysis of the presence or absence of CMV nucleic acid in a nucleic acid sample obtained from a wide variety of source. Non-limiting sources from which the nucleic acid may be extracted are serum, plasma, whole blood, urine or cerebrospinal fluid.

In reconstruction experiments, in which a known amount of virus was added to the specimen. It was found that the Q-PCR procedure performs equally well for serum and plasma specimens, regardless of the anticoagulant (citrate, heparin, or EDTA).

The methods of the invention comprise a highly sensitive diagnostic and quantitative assay for the detection of CMV by PCR. In one embodiment of the invention the amplified products (so-called amplimers) are detected following hybridisation with TBR-labelled probes and ECL detection by the QPCR System 5000 (Perkin Elmer). The lower limit of sensitivity of the D-PCR for CMV nucleic acid isolated from plasma or serum, is reached at about 25 CMV particles/ml. The presence of internal control DNA during extraction monitors for both extraction efficiency and PCR inhibitors, thus excluding a false negative diagnosis. The Q-PCR method of the invention reliably detects 4-fold differences in viral load in the range of 100 to 150,000 copies CMV/ml. The Q-PCR method is equally applicable for serum and plasma regardless of the anticoagulant.

In one embodiment of the invention is provided a method for the detection of human cytomegalovirus nucleic acid in a sample comprising subjecting said nucleic acid to a nucleic acid amplification method using a first and a second primer wherein the first primer or the second primer or both are chosen from a conserved region of human cytomegalovirus.

In a preferred embodiment of the invention one or both primers are chosen from a conserved region in exon 4 of the major immediate early gene of human cytomegalovirus. In a preferred embodiment of the invention the amplification is achieved by means of the polymerase chain reaction, however other methods of amplification such as RT-PCR or NASBA may also be used for the amplification step in the present invention. Preferably the amplified products, so-called amplimers, are separated from other components in the amplification reaction mixture. In a preferred embodiment of the invention one or more primers may be biotinylated. In a preferred embodiment of the invention the amplimers are detected with a probe. In a particularly preferred embodiment the amplimers are detected with a {Tris (2,2'-bipyridine) ruthenium(II) chelate} labelled probe. However, other methods for the detection of the amplimers may also be used such as gel-electrophoresis. Using other methods of detection of the amplimers may alter the sensitivity of the methods. In case gel-electrophoresis is used the sensitivity of detection is lower compared to detection with a {Tris (2,2'-bipyridine) ruthenium(II) chelate) labelled probe. In a preferred embodiment of the invention internal control (IC) nucleic acid capable of being amplified by using said first and second primer is added to the sample as a control for the nucleic acid extraction procedure. The amount of IC nucleic acid added to the sample may vary as long as the amount is sufficient to serve as a control for the nucleic acid extraction procedure. Preferably 35 IC nucleic acid molecules are added per 200 µl sample fluid. The sample fluid may be plasma or serum or other bodily fluids or fluids obtained after extraction of nucleic acid from tissues. The amount of IC DNA added to the sample may be increased to an amount suitable for the quantification of CMV nucleic acid in the sample. In the preferred embodiment where the amount of IC nucleic added to the sample is sufficient to serve as a control for the nucleic acid in a sample but not sufficient to allow the accurate quantification of CMV in the sample, extra IC nucleic molecules may be added in the amplification reaction to enable the accurate quantification of CMV nucleic acid in the sample. In a preferred embodiment of the invention said internal control nucleic acid is amplified with essentially the same efficiency as said virus nucleic acid. In a preferred embodiment of the invention this is achieved by designing the internal control nucleic acid such that the amplified part of the internal control DNA comprises essentially the same number of bases and essentially the same GC content as the amplified sequence of said human cytomegalovirus nucleic acid. In a preferred embodiment of the invention the internal control nucleic acid comprises at least one part of the sequence amplified in said virus and further comprises at least one sequence enabling the discrimination of the amplified products of said virus nucleic acid and said internal control nucleic acid. Discrimination of the amplimers of the internal control nucleic acid and the amplimers of the CMV nucleic acid may be achieved though a number of different methods. In a preferred embodiment of the invention the amplified products of said virus nucleic acid and said internal control nucleic acid are discriminated on the basis of a difference in restriction enzyme pattern, preferably through a difference in the pattern obtain using the restriction enzymes AocI, HhaI or both. In a preferred embodiment the internal control nucleic acid comprises the sequence in figure 9. In a particularly preferred embodiment of the invention the amount of human cytomegalovirus in a sample is quantitated.

In another aspect of the invention is provided a kit for the detection of nucleic acid of human cytomegalovirus comprising a first and a second primer wherein the first primer or the second primer or both are chosen from a conserved region of human cytomegalovirus. In a preferred embodiment of this aspect of the invention said first or said second primer or both are chosen from a conserved region in exon 4 of the major immediate early gene of human cytomegalovirus. In a preferred embodiment of the invention said kit further comprises a probe for the detection of the amplified product of said virus nucleic acid. In a particularly preferred embodiment of the probe in the kit comprises the sequence 5'TGA.AGG.TCT.TTG.CCC.AGT.ACA.TTC.T3' or the sequence 5'A.GAA.TGT.ACT.GGG.CAA.AGA.CCT.TCA3' or both. Said probe may be labelled preferably with {Tris (2,2'-bipyridine) ruthenium(II) chelate} to facilitate detection of the amplimer.

In a particularly preferred embodiment of the invention the kit further comprise an internal control nucleic acid capable of being amplified by using said first and second primer. In a preferred embodiment said internal control nucleic acid is capable of being amplified with essentially the same efficiency as said virus nucleic acid. Preferably this is property is obtained through the designing said internal control nucleic acid such that it comprises essentially the same number of bases and essentially the same GC content as the amplified sequence of said human cytomegalovirus. In a preferred embodiment said internal control nucleic acid comprises at least one part of the sequence amplified in said virus and further comprises at least one sequence enabling the discrimination of the amplified products of said virus nucleic acid and said internal control nucleic acid. In a preferred embodiment of the invention said kit further comprises a probe for the detection of the amplified product of said internal control nucleic acid. Preferably said probe comprises the sequence 5'CCC.TTT.ACA.TCT.TTC.TGA.AGT.AGG.G3' or the sequence 5'C.CCT.ACT.TCA.GAA.AGA.TGT.AAA.GGG3' or both. Said probe may be labelled preferably with {Tris (2,2'-bipyridine) ruthenium(II) chelate} to facilitate detection of the amplified product of said internal control nucleic acid. In a preferred embodiment of the kit of the invention the first primer comprises the sequence 5'CAC.TGG.CTC.AGA.CTT.GAC.AGA.CAC3'. In another preferred embodiment of the kit of the invention the second primer comprises the sequence 5'ACA.AGG.TGC.TCA.CGC.ACA.TTG.ATC3'. In yet another embodiment of the kit of the invention at least one primer is biotinylated.

In one aspect the invention provides a method for reliably and routinely detecting and/or quantifying human cytomegalovirus in a clinical sample suspected of containing human cytomegalovirus nucleic acid comprising
- adding to said sample a known amount of internal control nucleic acid;
- isolating nucleic acid from said sample;
- optionally adding to said sample another known amount of internal control nucleic acid;
- subjecting at least part of said nucleic acid in said sample to a nucleic acid amplification reaction using a first and a second primer;
- collecting any amplified products;
- performing a hybridisation reaction with part of said isolated amplified product using a probe specific for amplified cytomegalovirus nucleic acid;
- performing a hybridisation reaction with another part of said isolated amplified product using a probe specific for said internal control nucleic acid;
- detecting and/or quantifying amplified internal control nucleic acid and any amplified cytomegalovirus nucleic acid;
   wherein:
- said first primer comprises the sequence 5'CAC.TGG.CTC.AGA.CTT.GAC.AGA.CAC3' and said second primer comprises the sequence 5'ACA.AGG.TGC.TCA.CGC.ACA.TTG.ATC3';
- said internal control nucleic acid can be amplified by using said first and said second primer;
- said internal control nucleic acid is amplified with essentially the same efficiency as said virus nucleic acid;
- said internal control nucleic acid is added preferably in an amount effective for controlling said nucleic acid isolation procedure;
- said optionally added control nucleic acid is added preferably in an amount effective for quantifying the amount of cytomegalovirus nucleic acid in said sample.
In a preferred embodiment of this aspect of the invention at least one of said primers is biotinylated. Preferably a probe for the detection of any amplified cytomegalovirus nucleic acid comprises the sequence 5'TGA.AGG.TCT.TTG.CCC.AGT.ACA.TTC.T3' or its complementary sequence.
Preferably a probe for the detection of amplified internal control nucleic acid comprises the sequence 5'CCC.TTT.ACA.TCT.TTC.TGA.AGT.AGG.G3' or its complementary sequence. In one embodiment of the invention at least one probe is labelled with {Tris (2,2'-bipyridine) ruthenium(II) chelate}.
In one embodiment of the invention excess primers is removed after an amplification step, preferably through a Delta Y-A protocol.

### EXAMPLE 1

### Materials and methods

Chemicals and enzymes. Taq DNA polymerase (Amplitaq), Uracil-N-glycosylase, and streptavidin-coated magnetic beads were from Perkin Elmer (PE). Bovine serum albumin (BSA) was from Boehringer Mannheim. Human placental DNA and alpha casein (chromatographically purified to a purity of at least 85%, catalogue nr. C 6780) were from Sigma Chemical Company. Pools of human citrate plasma (ESDEP, Octapharma Pharmazeutica Produktions- gesellschaft m.b.H., Vienna, Austria) were obtained from the Central Laboratory for Blood Transfusion, Amsterdam, The Netherlands; this plasma is referred to as reference plasma. Plasma and serum specimens were obtained in Vacutainer tubes (Beckton Dickinson Systems, Meylan, France; K₃-EDTA, sodium citrate, lithium heparin).
Human cytomegalovirus. Sucrose density gradient purified Human cytomegalovirus strain AD 169 {lot nr. 80-165-1, 5.38 x 10⁹ viral particles/ml virus dilution buffer (10 mM TRIS.HCl,150 mM NaCl, 1 mM EDTA, pH 7.5), as determined electronmicroscopically by direct particle count which discriminated between full and empty particles} was obtained from Advanced Biotechnologies Inc (Columbia, MD, USA). According to the manufacturer the error in viral particle count was estimated to be ± 0.5 log. We therefore performed limiting dilution experiments for CMV DNA purified by the silica-guanidiniumthiocyanate procedure (5) from virus preparations followed by PCR; these experiments suggested that the actual amount of viral DNA was about 3 times higher than expected from the viral particle count assuming a 100% recovery of viral DNA (data not shown). CMV concentrations mentioned in this paper were based on this correction factor.
PCR. Primers (HPLC purified) were manufactured by Perkin Elmer; primers were diluted in TE buffer (10 mM TRIS.HCl,1 mM EDTA, pH 8.0) to 100 ng/ml and stored at -20°C. The primer pair used for amplification consisted of CMV-531 (5' ACA. AGG. TGC. TCA. CGC. ACA. TTG. ATC 3'; nucleotide positions 2034-2057) and Bio-CMV-1107 (5'CAC. TGG. CTC. AGA. CTT. GAC. AGA. CAC 3', 5' biotinylated; nucleotide positions 2588-2611); nucleotide numbering was according to Akrigg et al (1). This primer pair amplifies a 578 bp DNA fragment from exon 4 of the major immediate early gene of HCMV or a fragment of identical size and GC content from IC DNA. The primer pair was chosen in perfectly conserved areas of exon 4 of the major immediate early gene after alignment of 9 published sequences (1, 8, 36). The final reaction mixture (50 µl) contained 28 pmoles of each primer CMV-531 and Bio-CMV-1107, 2.5 U of Amplitag DNA polymerase, 0.5 U of Amperase (Uracil-N-glycosylase, Perkin Elmer), 5 µg BSA (Boehringer Mannheim), 10 mM TRIS-HCl (pH 8.3), 50 mM KCl, 3 mM MgCl₂, 200 µM each of dATP, dGTP and dCTP and 400 µM of dUTP (Perkin Elmer) and 25 µl (D-PCR) or 20 µl (Q-PCR) of isolated nucleic acid. PCR reactions were done in a Perkin Elmer 9600 thermocycler: 2 min at 50°C, 5 min at 95°C followed by 35 cycles each consisting of 20 sec at 95°C, 20 sec at 63°C, 1 min at 72°C; followed by 5 min at 72°C. No PCR products were obtained when DNA of other herpesviruses, all obtained from Advanced Biotechnologies Inc (Columbia, MD, USA) was used in the PCR reaction (not shown). These included HSV-1 (strain MacIntyre), HSV-2 (G-strain), EBV (strain B95-8), HHV-6 (strain Z-29), and VZV (strain Rod).
Internal Control (IC) DNA. A 597 bp amplimer was generated by PCR from CMV (AD169) DNA (Advanced Biotechnologies Inc, Columbia, MD, USA) with primers CMV-517, 5' GAT GAG GAG AGA GAC AAG GTG C 3' (nt 2021-2042) and CMV-1113, 5' CTC AGA CAC TGG CTC AGA CTT G 3' (nt 2596-2617). Nucleotide numbering was according to Akrigg et al (1). The amplimer was digested with *Aoc*I and a 227 bp fragment (nt 2021-2248) was purified from gel. The amplimer was also digested with A*lw*I and a 296 bp fragment (nt 2321-2617) was purified from gel. A double-stranded DNA sequence was obtained from *in vitro* synthesised single-stranded DNAs (5' phosphorylated, PE) by hybridisation of the complementary sequences 5'TGA CCT TAT CAG TGT AAT GAA CCG CCG CAT TGA GGA GAT CTG CAC CCT TTA CAT CTT TCT GAA GTA GGG G 3' and 5' CCC CCT ACT TCA GAA AGA TGT AAA GGG TGC AGA TCT CCT CAA TGC GGC GGT TCA TTA CAC TGA TAA GG 3'. This double-stranded DNA sequence contained DNA overhangs and was ligated to the *Aoc*I site of the 227 bp fragment and to the A*lw*I site of the 296 bp fragment generating a 597 bp fragment. This fragment was purified from gel and amplified by PCR using primers CMV-517 and CMV-1113 and the amplimer was cloned into a plasmid vector (PCRII, 3932 bp, Promega, Leiden, The Netherlands) resulting in a plasmid pCMV-marker which served as internal control (IC) DNA. Relative to the CMV sequence (1) the *Aoc*I site (5' CCTGAGG 3', nt 2246-2252), has been replaced by the sequence 5'CCTGACC 3'; similarly, the *Hha*I site (5' GCGC 3', nt 2269-2272) has been replaced by the sequence 5' CCGC 3'. In addition, the CMV sequence at nt 2292-2316 has been replaced by the sequence 5' CCC TTT ACA TCT TTC TGA AGT AGG G 3' to serve as a probe area. Restriction enzymes *Aoc*I and *Hha*I cleaved (uracil containing) CMV amplimers but did not cleave IC amplimers; *Hha*I and *Aoc*I sites were present in amplimers obtained by PCR from all (n = 80) clinical CMV isolates tested (not shown). The presence of *Aoc*I and *Hha*I sites in CMV DNA could therefore be used to discriminate between CMV and IC amplimers by gel electrophoresis. CMV and internal control amplimers could also be discriminated by probes specific for either CMV or IC specific areas (see below). These modifications allowed for discrimination between CMV and IC amplimers resulting from the PCR described in the above section, by the restriction enzymes *Hha*I and *Aoc*I and by probes specific for either CMV or IC specific areas (see below). pCMV-marker was purified from bacterial cultures as described (21) and the plasmid was linearised by *Hind*III digestion, purified by protocol Y/SC (5), and DNA was quantitated by UV absorption at 260 nm and stored in TE buffer (at 100 µg plasmid/ml) at -20°C. Dilutions of the linearised plasmid were made in TE buffer containing 20 ng human placenta DNA per µl; the presence of carrier DNA served to stabilise very dilute DNA preparations upon storage. Reference IC DNA contained 7 molecules of linearised plasmid (as determined by limiting dilution followed by PCR) and 20 ng of human placenta DNA per µl.
Preparation of lysis buffer L7A. Lysis buffer L7A was prepared by the addition of alpha casein to lysis buffer L6 {prepared as described previously (5)} to a final concentration of 1 mg/ml L6. L7A was stable for at least 2 months when stored at room temperature in the dark. Concentrated (50x) stock solutions (50 mg alpha casein/ml L6) were stored at -20°C and were stable for at least one year.
DNA purification. DNA was purified from 200 µl serum, plasma, cerebrospinal fluid (CSF) or from 50 µl whole blood as described previously (5) with the following modifications: 20 µl (rather than 40 µl) of size-fractionated silica particles (SC) were used in combination with 900 µl lysis buffer L7A, which contains alpha casein (rather than lysis buffer L6). DNA was eluted in 100 µl TE-buffer.
   In the diagnostic PCR (D-PCR) 25 µl of DNA eluate (corresponding to 50µl plasma or serum) was used as input for PCR. For quantitative PCR (Q-PCR), 20 µl of DNA eluate (corresponding to 40 µl of plasma or serum) and 5 µl Reference IC DNA (see below) was subjected to PCR.
   In the diagnostic and quantitative PCR, 5 µl of reference IC DNA (containing 7 copies of linearised pCMV-marker DNA and 20 ng of human placental DNA per µl TE buffer) was added to the lysis buffer/silica mixture. Next, the clinical specimen (200 µl serum or plasma) was added. The presence of 35 molecules of IC DNA during extraction served as a control for a lower limit of detection of 175 molecules per ml plasma or serum in the diagnostic procedure.
Removal of excess primers. Initial experiments revealed that the amount of biotin labelled primers used in the PCR reaction exceeded the biotin binding capacity of the streptavidin-coated magnetic beads. A protocol (Protocol Delta Y-A) was developed to remove excess primers. This protocol is a significantly shortened version of the previously described procedure for purification of nucleic acids from clinical materials (5). In this protocol, 40 µl of PCR product were added to a mixture of 1 ml lysis buffer L6 and 20 µl SC; after vortexing, the tubes were left at ambient temperature for 10 min and subsequently centrifuged (1 min at approx. 12,000 x g). The supernatant was removed by suction and the tube was centrifuged again (1 min at approx. 12,000 x g) after which the supernatant was carefully removed by suction. The silica pellet was washed once with 1 ml of acetone and after centrifugation (1 min at 12,000 x g) the supernatant was carefully removed by suction. Together, these steps removed most of the GuSCN present in the lysis buffer which would otherwise interfere with hybridisation. Subsequently, the silica pellets were dried in an Eppendorf heatblock (10 min at 56°C, open lids). DNA was eluted in 100 µl 1x PCRII buffer (Perkin Elmer, 10 mM TRIS-HCl, pH 8.3, 50 mM KCl) for 10 min at 56°C followed by vortexing and subsequent centrifugation for 2 min at approx. 12,000 x g. The supernatant contained the purified PCR product. The purified PCR product was subsequently used for hybridisation. About 24 samples could be handled within an hour, yields were high (near 100% for both IC and CMV amplimers as judged from EthBr stained gels) and primers were efficiently removed (data not shown). This particular protocol was chosen from several other shortened versions of the Silica-GuSCN procedure (5) because it gave the lowest variation (coefficient of variation 5%) when the purified amplimers were subsequently used in hybridisation and ECL measurements (data not shown).
Hybridisation and ECL measurement. For D-PCR, purified PCR product (30 µl) was used directly in the hybridisation reaction. For Q-PCR, purified PCR product was diluted 5 times in 1x PCR II buffer and 30 µl of this dilution were used in the hybridisation reaction. Hybridisation was in a total volume of 50 µl. To 30 µl of the purified PCR product 20 µl of TBR-labelled probe (1 ng/µl; 1.3 pmoles, either CMV- or IC-specific) was added and hybridisation was done in a Perkin Elmer 9600 thermocycler (2 min at 95°C followed by 5 min at 56°C). Subsequently, 10 µl of streptavidin coated magnetic beads (PE) were added at ambient temperature and the mixture was incubated for 15 min at 56°C. Forty µl of the beads-hybrids suspension were added to 400 µl QPCR assay buffer (Perkin Elmer) and the ECL signal, expressed in luminosity units, was measured by the QPCR System 5000 according to the instructions of the manufacturer (Perkin Elmer). In this device, excess TBR-labelled probe is automatically removed by washing and the amount of labelled hybrids is determined after excitation by applying an electric field.
   TBR-labelled probes were diluted in 1x PCRII buffer (Perkin Elmer, 10 mM TRIS.HCl pH 8.3, 50 mM KCl) to 1 ng/µl and stored at -20°C. TBR-labelled probes were TBR-CMV-1 (CMV-specific probe: 5'-TGA. AGG. TCT. TTG. CCC. AGT. ACA. TTC. T 3'; nucleotide positions 2292-2316) and TBR-CMV-2 (IC-specific probe: 5' CCC. TTT. ACA. TCT. TTC. TGA. AGT. AGG. G 3'); Both probes contained a single TBR-label at the 5' end. No cross hybridisation was observed for IC-specific and CMV-specific probes (data not shown). TBR-CMV-1 was chosen in a highly conserved area of exon 4 of the major immediate early gene after alignment of 9 published sequences (1, 8, 36).
Criteria for the D-PCR. A signal of >80 LU (= 4 x mean background signal for either probe) was considered to be positive. In the D-PCR a serum or plasma specimen was considered positive for CMV DNA if >80 LU were measured with the CMV probe, regardless of the IC results (at loads of > 50,000 copies CMV/ml, IC results were near background in the D-PCR). A serum or plasma specimen was considered negative for CMV DNA if < 80 LU were measured with the CMV probe and, additionally, IC DNA was detected at >80 LU. If both CMV DNA and IC DNA were not detected (<80 LU), the test was considered false-negative and was rejected.
   In the D-PCR three controls were included in the DNA extraction, one positive control (a reference plasma containing 400 copies CMV/ml), and two negative controls. The first negative control contained human chromosomal DNA (100 ng) and 35 molecules of IC DNA, and served as a control for the entire QPCR procedure and as a control for the sensitivity of the procedure. The second negative control contained human chromosomal DNA (100 ng) only and should give negative results with both probes.
Algorithm for quantitation in the Q-PCR. The algorithm assumes ideal circumstances for each of the steps of the procedure and would be valid if 1) recovery of CMV DNA and IC DNA from plasma were 100% and, 2) if CMV and Internal Control DNA were amplified with equal efficiencies and, 3) if CMV and IC amplimers were purified with equal efficiencies and, 4) if hybridisation with either probe was quantitative and, 5) if bead capture and measurement -by ECL- of the amount of hybrids were quantitative. In the Q-PCR, DNA was purified from 200 µl plasma or serum together with 35 molecules of IC DNA, and DNA was eluted in 100 µl TE buffer. Twenty µl of DNA (derived from 40 µl of plasma) were subjected to Q-PCR in the presence of an additional 35 molecules of IC DNA which were present in the PCR mastermix, and ECL signals obtained after hybridisation with TBR-labelled probes were determined as described above. Background signals (mean 20 LU, cv=40%) for CMV-specific and IC-specific probes were determined for 23 plasmas previously shown to be CMV negative. After background correction, the ratio of (CMV-specific signal)/(IC-specific signal), (R), was calculated and the copy number of CMV per ml plasma was calculated by amplifying R with a factor 1050. This factor was reached from two separate factors (35+7) x 25, which represent the number of IC DNA molecules present during the PCR (sum of co-extracted and added IC DNA molecules), and a factor (of 25) to reach the copy number per ml plasma respectively. Thus, the clear cut algorithm "copies CMV/ml = R x 1050" was used throughout this paper for all Q-PCR experiments.
   It appears that Perkin-Elmer no longer supplies the QPCR System 5000. Reagents (assay buffer and cell cleaner) and equipment are now available from the original manufacturer (IGEN International, Inc., 16020 Industrial Drive, Gaithersburg, Maryland 20877, USA); TBR-labelled probes and streptavidin-coated magnetic beads (4.5 µm streptavidin Dynabeads suspended in assay buffer) can still be obtained from P.E.

### EXAMPLE 2

Sensitivity of amplimer detection by TBR-labelled probes relative to ethidium bromide (EthBr) staining. The use of TBR-labelled probes and measurement of the amount of hybrids by ECL provides an attractive alternative for agarose gel electrophoresis and EthBr staining for detection of PCR products, not only because of specificity control and quantitative aspects, but also because of the significantly higher sensitivity relative to EthBr staining (23). Comparison of EthBr staining and ECL detection of the 587 bp amplimer showed a lower level of EthBr detection of about 48 x 10⁸ molecules (3000 pg), whereas the lower limit of ECL detection was reached at about 3 x 10⁸ molecules (200 pg). Thus for an amplimer of this size (578 bp) ECL detection was about 15-fold more sensitive than EthBr staining (data not shown). When a constant amount of beads-hybrid complexes was used as input for ECL measurement, the coefficient of variation (cv) in ECL signals was 2 %. When a constant amount of PCR product was used as input for primer removal followed by hybridisation, beads binding, and ECL measurement, variation in ECL signals was about 5% (data not shown).

Recovery of DNA from plasma and lower limit of detection. We have previously reported on a rapid and simple method for the purification of nucleic acids from clinical specimens (Silica-GuSCN procedure) (5). The basic purification procedure is now widely used for DNA isolation but as yet, no information is available about DNA yields at very low inputs. Recently, the performance of an adaptation of the basic procedure, in which the original lysis buffer is supplemented with alpha casein, has been evaluated using samples containing high concentrations of DNA. All DNA extractions described in this manuscript were done with this novel lysis buffer. However, also the lysis buffer without alpha casein may also be used with essentially similar results.

To study DNA recovery from plasma at very low DNA concentrations, reference plasma was supplemented with 4 molecules of IC DNA per 200 µl plasma, DNA extractions were done in 20-fold from 200 µl samples, and one quarter of the extracted DNA was used as input for the PCR. At 100% extraction efficiency this would result in the presence of a single molecule of IC DNA per PCR. In such cases Poisson statistics predicts 63% of the reactions to be positive (11). In Figure 1A it is shown that 50% of the PCR reactions were positive suggesting that DNA recovery at this extremely low input was near 100%. Control extractions (reference plasma only) were done in four-fold and were all negative (Figure 1A). As a control for the concentration of the IC DNA preparation which was used to prepare the IC DNA/plasma mixtures, the same IC DNA preparation was used *directly* in 10 PCR reactions at an input expected to contain a mean of 1 molecule of IC DNA per PCR reaction. In this case PCR was positive in 40% of the PCR reactions, confirming the IC DNA concentration (Figure 1B). This data shows that even at extremely low DNA inputs (4 molecules / 200 µl plasma), DNA was recovered with very high (near 100%) yields. Results of several other experiments, not shown here, consistently showed similar high DNA yields.

To study the lower limit of CMV DNA detection in plasma, reference plasma was supplemented with CMV particles to a concentration of 100 copies CMV/ml plasma and serial twofold dilutions were made in the same plasma. DNA was extracted from 200 µl samples together with 35 molecules of IC DNA and one-fifth of the eluates was subjected to D-PCR. Figure 2 shows that at extraction inputs as low as 5 viruses per 200 µl plasma (resulting in a mean of 1 copy CMV DNA in the PCR reaction at 100% extraction efficiency), the assay was still positive for CMV DNA in 4 out of 6 reactions (which is in accord with the number of expected positives, at 100% extraction efficiency, by Poisson distribution (11). The negative results (i.e. ECL signals <80 LU) were true negatives since ECL signals for co-extracted IC DNA (35 molecules/200 µl plasma) were all positive (>80 LU). Together this data suggests that CMV DNA was recovered with high yields even at viral loads as low as 25 copies CMV/ml plasma.
Diagnostic assay. In the D-PCR, co-extraction of IC DNA (35 molecules) with serum or plasma DNA served as internal control for the performance of the entire diagnostic procedure, including DNA purification, and as a control for the sensitivity of the procedure. In the D-PCR three controls were included, one positive control (a reference plasma containing about 400 copies CMV/ml), and two negative controls. The first negative control contained human chromosomal DNA and 35 molecules of IC DNA, and served as a control for the entire QPCR procedure and as a control for the sensitivity of the procedure. The second negative control contained human chromosomal DNA only and should give negative results with both probes. Figure 3 shows an example of the diagnostic assay in which 8 consecutive serum specimens from a renal transplant patient (CMV seronegative, with a seropositive kidney donor) were tested for the presence of CMV DNA. Initial serum specimens (days 0 and 10 after transplantation) were CMV DNA negative, followed by a period in which CMV DNA was detected (days 38-66 after transplantation). During this period seroconversion for CMV was observed, the patient presented with hepatitis and fever and was treated with ganciclovir. In the last specimen, obtained 70 days after transplantation, CMV DNA was no longer detected.
   In the D-PCR, DNA was purified from 200 µl of a plasma or serum specimen together with 35 molecules of IC DNA by a modification of the silica-GuSCN procedure (5). Next, purified DNA was subjected to a single round of PCR with a single primer pair (of which one primer was biotinylated) in the presence of Uracil-N-glycosylase and dUTP to prevent false-positive reactions due to amplimer carry-over. In the case of a CMV positive specimen, this gave rise to a mixture of IC and CMV amplimers of identical length (587 bp) and GC content. CMV and IC amplimers could be discriminated by hybridisation with TBR-labelled probes followed by ECL detection in the QPCR System 5000 (Perkin Elmer). The use of TBR-labelled probes and detection by ECL was about 15-fold more sensitive than EthBr staining of agarose gels, with a lower limit of detection of about 3 x 10⁸ molecules (200 pg) of amplimer. This lower limit of detection by ECL is in accord with previous investigations (23, 39). The presence of a low amount of IC DNA during extraction served as a control for the entire diagnostic procedure, including DNA extraction, and as a control for the presence of PCR inhibitors, thus ruling out false-negative reactions. The lower limit of sensitivity of the D-PCR assay was reached at about 25 CMV particles/ml plasma or serum. The presence of 35 molecules of IC DNA during extraction from 200 µl plasma or serum allowed for the conclusion that a specimen found negative for CMV DNA but positive for IC DNA would contain less than 175 copies CMV/ml plasma or serum.
Quantitative assay. Reference plasma was supplemented with CMV to a concentration of 150.000 copies/ml, and serial three-fold dilutions were made in the same plasma to reach concentrations down to 206 copies CMV/ml. CMV DNA was extracted from 200 µl samples together with 35 molecules of IC DNA, as in the diagnostic assay. One fifth of the extracted DNA was used in the PCR in the presence of an additional amount (35 molecules) of IC DNA. This additional DNA served to dampen variation due to Poisson distribution since quantitation would otherwise rely on the quantitative detection of only 7 molecules of IC DNA. This additional IC DNA also served to extend the dynamic range of the Q-PCR for high viral loads. Figure 4A shows the ECL signals obtained by Q-PCR after hybridisation with the CMV-specific probe and the IC-specific probe for such a plasma dilution series. In this figure it can be seen that with increasing virus load, the amount of CMV-specific hybrids increased until a plateau level was reached. This plateau level was a reflection of the amount of amplimers present at the plateau phase of the PCR reaction (data not shown). At low CMV concentrations, IC ECL signals were unaffected by increasing viral loads, at higher viral loads IC ECL signals decreased. This decrease was due to the fact that the PCR reached plateau earlier at higher viral loads. When this plateau is reached during PCR, IC amplification will also enter a plateau phase at a level determined by the viral load. In other words, the initial ratio (CMV DNA/IC DNA) present at the start of the PCR reaction will be maintained throughout PCR and plateau phase. If the amounts of CMV and IC DNA were accurately measured in the next steps of the procedure the CMV/IC ratio would be a straight line with a slope corresponding to a serial 3-fold dilution. Figure 4B shows that the CMV/IC ratios obtained for this plasma dilution series were in good accordance with the expected ratios. By the algorithm described in Materials this ratio was amplified by a constant factor (of 1050) to calculate the viral load expressed as copies CMV/ml (Figure 4C). Expected and calculated values were in good accordance for viral loads from 206 to 150,000 copies CMV/ml.
Variation. Inter-assay variation of the Q-PCR was determined by repeating the experiment described above another six times on different days over a period of 3 months. Figure 5 summarises the results obtained from these experiments. To determine the intra-assay variation, each of the plasma dilutions was tested in 8-fold in the Q-PCR. Negative controls (reference plasma) were included in each run. The data, summarised in table 1, suggests that inter-assay variation and intra assay variation were similar with a mean coefficient of variation of 25%.
   Another important question in quantitation of viral load was whether serum specimens, obtained from different patients and containing the same viral loads would give the same results by Q-PCR (interspecimen variation). To answer this question, 10 serum specimens obtained from different CMV-seronegative subjects (including 1 renal transplant patient and 9 chronic fatigue syndrome patients) were supplemented with CMV to 15,000 and 1667 copies/ml or were left unseeded. The unseeded specimens were tested by D-PCR and were found to be negative for CMV DNA (not shown). For the ten sera containing 15,000 copies CMV DNA/ml, a mean value of 15,065 copies CMV DNA/ml was calculated with a coefficient of variation of 25%. A similar coefficient of variation (23%) was obtained for the ten sera containing 1667 copies CMV DNA/ml with a calculated mean of 2363 copies CMV/ml. Since this variation was the same as that obtained in inter- and intra assay variation, it was concluded that the Q-PCR reliably measured viral loads, independently of serum donor. Similar results as described above were obtained when plasma specimens rather than serum specimens were tested by Q-PCR (data not shown).
   To study the contribution of variation in DNA extraction efficiency to the overall variation of the Q-PCR, the DNA extracts from the ten serum specimens supplemented with CMV to 1,667 copies/ml mentioned above, were pooled and the viral load was determined In ten-fold for this pooled DNA. The mean calculated load was 2527 copies CMV/ml with a variation coefficient of 16%. This data suggests that variation in DNA extraction efficiency contributed only 7% to the total observed variation of 23% as calculated for the separate extractions followed by Q-PCR.
Quantitative data obtained by Q-PCR. The sequential serum specimens from the renal transplant patient analysed above in the D-PCR (Figure 3) were also tested in the Q-PCR. Figure 6 shows that after an initial CMV negative phase, a viral load of 75 copies/ml was observed at day 38 which increased rapidly to a level of 1275 copies/ml at day 41 and further increased to 6025 copies/ml at day 47 after transplantation. At this time, the patient presented with fever and elevated liver enzymes, seroconversion (for both IgG and IgM) for CMV was observed and ganciclovir therapy was started at day 47. Serum CMV DNA levels decreased to undetectable levels at day 66. CMV culture was first positive for buffy coat cells obtained at day 38 (after 24 days of culture) and remained negative thereafter.
   Serum and plasma are equivalent substrates for the Q-PCR. To establish whether plasma specimens, obtained by different anticoagulants (heparin, EDTA, or citrate), and serum were equivalent substrates for the Q-PCR, serum and plasma specimens from 4 healthy subjects were seeded with constant amounts of CMV (to 15,000, 1000, and 185 copies CMV/ml) and subjected to QPCR. Unseeded specimens were negative for CMV DNA (not shown). The data presented in Figure 7 shows that viral loads determined by Q-PCR were not significantly different for serum and plasma (repeated measures analysis of variance, p=0.65, p=0.21 and p=0.45 for 15,000, 1000 and 185 copies CMV/ml resp.). This data shows that CMV loads can be reliably determined by Q-PCR in serum and plasma specimens irrespective of the anticoagulant.
   CMV loads in patient serum specimens may be significantly lower than loads observed in plasma. When paired serum and plasma specimens from CMV D-PCR positive patients were tested by Q-PCR, it was observed that serum DNA levels were usually significantly lower than plasma DNA levels (Table 2). An example is shown in Figure 8A in which CMV loads in citrate plasma, EDTA plasma and serum were determined in duplicate by Q-PCR. Mean serum load was about 25 copies/ml whereas the mean plasma load was 489 copies/ml. To show that this particular serum specimen was a good substrate for Q-PCR, CMV was added to about 750 copies/ml. As a control, citrate plasma, EDTA plasma, and serum from a healthy, CMV DNA-negative, volunteer was also seeded with CMV to the same level. Q-PCR performed in duplicate for these specimens showed that similar loads were obtained (Figure 8B), suggesting that the observed differences between serum and plasma loads in patient specimens were not an artefact of the Q-PCR procedure.

### Refefences

1 Akrigg, A., G.W.G. Wilkinson and J.D. Oram. 1985. The structure of the major immediate early gene of human cytomegalovirus strain AD169. Virus Research 2:107-121.
2 Allen, R.D., P.E. Pellett, J.A. Stewart, and M. Koopmans. 1995. Nonradioactive PCR-enzyme-linked immunosorbent assay method for detection of human cytomegalovirus DNA. J. Clin. Microbiol. 33:725-728.
3 Aspin, M.A., G.M. Gallez-Hawkins, T.D. Giugni, B. Tegtmeier, D.J. Lang, G.M. Schmidt, S.J. Forman, and J. Zaia. 1994. Comparison of plasma PCR and bronchoalveolar lavage fluid culture for detection of cytomegalovirus infection in adult bone marrow transplant recipients. J. Clin. Microbiol. 32:2266-2269.
4 Boom, R., J.L. Geelen, C.J. Sol, A.K. Raap, R.P. Minnaar, B.P. Klaver and J. van der Noordaa. 1986. Establishment of a rat cell line inducible for the expression human cytomegalovirus immediate early gene products by protein synthesis inhibition. J. Virol. 58:851-859.
5 Boom, R., C.J.A. Sol, M.M.M. Salimans, C.L. Jansen, P.M.E. Wertheim-van Dillen, and J. van der Noordaa. 1990. Rapid and simple method for purification of nucleic acids. J. Clin. Microbiol. 28:495-503.
6 Brytting, M., W. Xu, B. Wahren, and V. Sundqvist. 1992. cytomegalovirus DNA detection in sera from patients with active cytomegalovirus infections. J. Clin. Microbiol. 30:1937-1941.
7 Chan, A., J. Zhao, and M. Krajden. 1994. Polymerase chain reaction kinetics when using a positive internal control target to quantitatively detect cytomegalovirus target sequences. J. Virol. Meth. 48:223-236.
8 Chou, S. Effect of interstrain variation on diagnostic DNA amplification of the cytomegalovirus major immediate-early region. 1992. J. Clin. Microbiol. 30:2307-2310.
9 Cross, N.C.P. 1995. Quantitative PCR techniques and applications. Br. J. Haematology 89:693-697.
10 Cunningham, R., A. Harris, A. Frankton, and W. Irving. 1995. Detection of cytomegalovirus using PCR in serum from renal transplant recipients. Clinical Pathology 48:575-577.
11 Diaco, R. 1995. Practical considerations for the design of quantitative PCR assays. In: PCR strategies, pp 84-108. M.A. Innis, D.H Gelfand, and J.J. Sninsky (eds). Academic Press Inc.
12 Dickover, R.E., R.M. Donovan, E. Goldstein, S. Dandekar, C.E. Bush, and J.R. Carlson. 1990. Quantitation of human immunodeficiency virus DNA by using the polymerase chain reaction. J. Clin. Microbiol. 28:2130-2133.
13 Dodt, K.K., P.H. Jacobsen, B. Hofmann, C. Meyer, H.J. Kolmos, P. Skinhoj, B. Norrild, and L. Mathiesen. 1997. Development of cytomegalovirus (CMV) disease may be predicted in HIV-infected patients by CMV polymerase chain reaction and the antigenemia test. AIDS 11:F21-F28.
14 Drouet, E., S. Michelson, G. Denoyel, and R. Colimon. 1993. Polymerase chain reaction detection of human cytomegalovirus in over 2000 blood specimens correlated with virus isolation and related to urinary virus excretion. J. Vir. Methods 45: 259-276.
15 Freymuth, F., E. Gennetay, J. Petitjean, G. Eugene, B. Hurault de Ligny, J-P. Ryckelynck, C. Legoff, P. Hazera, and C. Bazin. 1994. Comparison of nested PCR for detection of DNA in plasma with pp65 leukocytic antigenemia procedure for diagnosis of human cytomegalovirus infection. J. Clin. Microbiol. 32:1614-1618.
16 Gallez-Hawkins, G.M., B.R. Tegtmeier, A. ter Veer, J.C. Niland, S.J. Forman, and J.C. Zaia. 1997. Evaluation of a quantitative plasma PCR plate assay for detecting cytomegalovirus infections in marrow transplant recipients. J. Clin. Microbiol. 35:788-790.
17 Gerna, G., F. Baldanti, A. Sarasini, M. Furione, E. Percivalle, M.G. Revello, D. Zipeto, D. Zella, and the Italian Foscarnet study group. 1994. Effect of foscarnet induction treatment on quantitation of human cytomegalovirus (HCMV) DNA in peripheral blood polymorphonuclear leukocytes and aqueous humor of AIDS patients with HCMV retinitis. Antimicrobial Agents and Chemotherapy 38:38-44.
18 Gerna,G., M. Furione, F. Baldanti,and A. Sarasini. 1994. Comparative quantitation of human cytomegalovirus DNA in blood leucocytes and plasma of transplant and AIDS patients. J. Clin. Microbiol. 32:2709-2717.
19 Hansen, K.K., A. Ricksten, B. Hofmann, B. Norrild, S. Olofsson, and L. Mathiesen. 1994. Detection of cytomegalovirus DNA in serum correlates with clinical cytomegalovirus retinitis in AIDS. J. Infect. Dis. 170:1271-1274.
20 Hebart, H., C. Miller, J. Loffler, G. Jahn, and H. Einsele. 1996. Monitoring of CMV infection: a comparison of PCR from whole blood, plasma-PCR, pp65-antigenemia and virus culture in patients after bone marrow transplantation. Bone Marrow Transpl. 17:861-868.
21 Ish-Horowicz, D., and J.F. Burke. 1981. Rapid and efficient cosmid cloning. Nucleic Acids Res. 9: 2989-2998-.37.
22 Ishigaki, S., M. Takeda,, T. Kura, N. Ban, T. Satoi, S. Sakamaki, N. Watanabe, Y. Kogho, and Y. Nitsu. 1991. cytomegalovirus DNA in the sera of patients with cytomegalovirus pneumonia. Br. J. Haematol. 79:198-204.
23 Kenten, J.H., S. Gudibande, J. Link, J.J. Willey, B. Curfman, E.O. Major, and R.J. Massey. 1992. Improved electrochemiluminescent label for DNA probe assays: rapid quantitative assays of HIV-1 polymerase chain reaction products. Clin. Chem. 38:873-879.
24 Laue, T., T. Mertenskötter, T. Grewing, O. Degen, J. van Lunzen, M. Dietrich, and H. Schmitz. 1997. Clinical significance of qualitative human cytomegalovirus (HCMV) detection in cell-free serum samples in HIV-infected patients at risk for HCMV disease. AIDS 11:1195-1196.
25 Nelson, C.T., A.S. Istas, M.K. Wilkerson, and G.J. Demmler. 1995. PCR detection of cytomegalovirus DNA in serum as a diagnostic test for congenital cytomegalovirus infection. J. Clin. Microbiol. 33:3317-3318.
26 Nigro, G., A. Krzysztofiak, G.C. Gattinara, T. Mango, M. Mazzocco, M.A. Porcaro, S. Provvedi, and J.C. Booth. 1996. Rapid progression of HIV disease in children with cytomegalovirus DNAemia. AIDS 10:1127-1133.
27 Nolte, F.S., R.K. Emmens, C. Thurmond, P.S. Mitchell, C. Pascuzzi, S.M. Devine, R. Saral, and J.R. Wingard. 1995. Early detection of human cytomegalovirus viremia in bone marrow transplant recipients by DNA amplification. J. Clin. Microbiol. 33:1263-1266.
28 Patel, R., T.F. Smith, M. Espy, R.H. Wiesner, R.A.F. Krom, D. Portela, and C.V. Paya. 1994. Detection of cytomegalovirus DNA in sera of liver transplant recipients. J. Clin. Microbiol. 32:1431-1434.
29 Patel, R., T.F. Smith, M. Espy, D. Portela, R.H. Wiesner, R.A.F. Krom, and C.V. Paya. 1995. A prospective comparison of molecular diagnostic techniques for the early detection of cytomegalovirus in liver transplant recipients. J. Infect. Dis. 171:1010-1014.
30 Rasmussen, L., S. Morris, D. Zipeto, J. Fessel, R. Wolitz, A. Dowling, and T.C. Merigan. 1995. Quantitation of human cytomegalovirus DNA from peripheral blood cells of human immunodeficiency virus-infected patients could predict cytomegalovirus retinitis. J. Infect. Dis. 171:177-182.
31 Saiki, R.K., D.H. Gelfand, S. Stoffel, S.J. Scharf, R. Higuchi, G.T. Horn, K.B. Mullis, and H.A. Erlich. 1988. Primer-directed enzymatic amplification of DNA with a thermostable DNA polymerase. Science 239: 487-491.
32 Schmidt, C.A., H. Oettle, R. Peng, P. Neuhaus, G. Blumhardt, R. Lohmann, F. Wilborn, K. Osthoff, J. Oertel, H. Timm, and W. Siegert. 1995. Comparison of polymerase chain reaction from plasma and buffy coat with antigen detection and occurrence of immunoglobulin M for the demonstration of cytomegalovirus infection after liver transplantation. Transplantation 59:1133-1138.
33 Shinkai, M., S.A. Bozette, W. Powderly, P. Frame, and S.A. Spector. 1997. utility of urine and leukocyte cultures and plasma DNA polymerase chain reaction for identification of AIDS patients at risk for developing human cytomegalovirus disease. J. Infect. Dis. 175:302-308.
34 Smith, I.L., M. Shinkai, W.R. Freeman, and S.A. Spector. 1996. Polyradiculopathy associated with ganciclovirresistant cytomegalovirus in an AIDS patient: phenotypic and genotypic characterization of sequential virus isolates. J. Infect. Dis. 173:1481-1484.
35 Spector, S.A., R. Merrill, D. Wolf, and W.M. Dankner. 1992. Detection of human cytomegalovirus in plasma of AIDS patients during acute visceral disease by DNA amplification. J. Clin. Microbiol. 30:2359-2365.
36 Stenberg, R.M., D.R. Thomsen, and M.F. Stinski. 1984. Structural analysis of the major immediate early gene of human cytomegalovirus. J. Virol. 49:190-199.
37 Tokimatsu, I., T. Tashiro, and M. Nasu. 1995. Early diagnosis and monitoring of human cytomegalovirus pneumonia in patients with adult T-cell leukemia by DNA amplification in serum. Chest 107:1024-1027.
38 Wolf, D.G., and S.A. Spector. 1993. Early diagnosis of human cytomegalovirus disease in transplant recipients by DNA amplification in plasma. Transplantation 56:330-334.
39 Yu, H., J.G. Bruno, T. Cheng, J.J. Calomiris, M. T. Goode, and D. L. Gatto-Menking. 1995. A comparative study of PCR product detection and quantitation by electrochemiluminescence and fluorescence. J. Biolum. Chemilumin. 10:239-245.
40 Zaia, J.A., G.M. Gallez-Hawkins, B.R. Tegtmeier, A. ter Veer, X. Li, J.C. Niland, and S.J. Forman. 1997. Late cytomegalovirus disease in marrow transplantation is predicted by virus load in plasma. J. Infect. Dis. 176:782-785.
41 Zipeto, D., F. Baldanti, D. Zella, M. Furione, A. Cavicchini, G. Milanesi, and G. Gerna. 1993. Quantification of human cytomegalovirus DNA in peripheral blood polymorphonuclear leukocytes of immunocompromised patients by the polymerase chain reaction. J. Virological Methods 44:45-56.
42 Zipeto, D., S. Morris, C. Hong, A. Downing, R. Wolitz, T.C. Merigan, and L. Rasmussen. 1995. Human cytomegalovirus (CMV) DNA in plasma reflects quantity of CMV DNA present in leukocytes. J. Clin. Microbiol. 33:2607-2611.

**Table 1**

| **Copies CMV/ml plasma** | **Inter assay variation mean (% CV)** | **Intra assay variation mean (% CV)** |
|---|---|---|
| 150,000 | 151,908 (18) | 124,791 (36) |
| 50,000 | 51,346 (29) | 48,258 (39) |
| 16,665 | 18,213 (28) | 15,423 (27) |
| 5,556 | 6,720 (14) | 8,128 (8) |
| 1,851 | 3,231 (38) | 2,790 (24) |
| 617 | 940 (16) | 884 (19) |
| 206 | 336 (29) | 308 (22) |

**Table 2**

| Subject | Copies CMV/ml Heparin plasma | Copies CMV/ml Citrate plasma | Copies CMV/ml EDTA plasma | Copies CMV/ml Serum |
|---|---|---|---|---|
| A. (RTX) | NA | 446 | 497 | 31 |
| | NA | 463 | 549 | 20 |
| B.(RTX) | 57 | 160 | 159 | neg |
| | 59 | 146 | 137 | neg |
| C.(RTX) | NA | 351 | 689 | neg |
| | NA | 802 | 668 | 58 |

| D.(ABMT) | | | | |
|---|---|---|---|---|
| day 49 | NA | NA | 287 | 166 |
| day 52 | NA | NA | 631 | 387 |
| day 54 | NA | NA | 3872 | 1121 |
| E.(AIDS) | 5518 | 2313 | 10649 | 809 |
| | 7095 | 6774 | 9644 | 580 |
| F.(RTX) | NA | NA | 300 | neg |

| G(RTX) | | | | |
|---|---|---|---|---|
| day 32 | NA | NA | 1715 | 75 |
| day 41 | NA | NA | 1636 | 83 |

## Claims

1. A method for the detection of human cytomegalovirus nucleic acid in a sample comprising subjecting said nucleic acid to a nucleic acid amplification method using at least two primers chosen from a conserved region in exon 4 of the major immediate early gene of human cytomegalovirus and wherein a first primer comprises the sequence 5'CAC.TGG.CTC.AGA.CTT.GAC.AGA.CAC3' and a second primer comprises the sequence 5'ACA.AGG.TGC.TCA.CGC.ACA.TTG.ATC3'.

2. A method according to claim 1 wherein amplification of said nucleic acid is achieved by means of PCR, RT-PCR or NASBA.

3. A method according to claim 1 or claim 2 wherein at least one primer is biotinylated.

4. A method according to anyone of the claims 1-3, wherein amplified product of said virus nucleic acid is detected with a probe.

5. A method according to claim 4 wherein said probe comprises the sequence 5'TGA.AGG.TCT.TTG.CCC.AGT.ACA.TTC.T3' or comprises the sequence 5'A.GAA.TGT.ACT.GGG.CAA.AGA.CCT.TCA3'.

6. A method according to claim 4 or claim 5 wherein said probe is labelled with {Tris (2,2'-bipyridine) ruthenium(II) chelate}.

7. A method according to anyone of the claims 1-6, further comprising adding to a sample an internal control nucleic acid capable of being amplified by using said first and second primer.

8. A method according to claim 7 wherein said internal control nucleic acid is amplified with essentially the same efficiency as said virus nucleic acid.

9. A method according to claim 7 or claim 8, wherein the sequence of said internal control nucleic acid comprises essentially the same number of bases and essentially the same GC content as the amplified sequence of said human cytomegalovirus nucleic acid.

10. A method according to anyone of the claims 7-9, wherein said internal control nucleic acid comprises at least one part of a sequence amplified in said virus and further comprises at least one sequence enabling discrimination of amplified products of said virus nucleic acid and said internal control nucleic acid.

11. A method according to anyone of the claims 1-10, wherein said internal control nucleic acid comprises the sequence of figure 9.

12. A method according to anyone of the claims 7-11, wherein amplified products of said virus nucleic acid and said internal control nucleic acid are discriminated on the basis of a difference in restriction enzyme pattern.

13. A method according to anyone of the claims 7-12, wherein amplified products of said virus and said internal control nucleic acids are discriminated by a difference in a restriction enzyme pattern of AocI, HhaI or both.

14. A method according to anyone of the claims 7-13, wherein amplified product of said internal control nucleic acid is detected with a probe.

15. A method according to claim 14, wherein said probe comprises the sequence 5'CCC.TTT.ACA.TCT.TTC.TGA.AGT.AGG.G3' or comprises the sequence 5'C.CCT.ACT.TCA.GAA.AGA.TGT.AAA.GCG3'.

16. A method according to claim 14 or claim 15, wherein said probe is labelled with {Tris (2,2'-bipyridine) ruthenium(II) chelate}.

17. Quantitation of the amount of human cytomegalovirus in a sample by using a method according to anyone of the claims 1-16.

18. A kit for the amplification of nucleic acid of human cytomegalovirus in a sample, comprising at least two primers chosen from a conserved region in exon 4 of the major immediate early gene of human cytomegalovirus and wherein a first primer comprises the sequence 5'CAC.TGG.CTC.AGA.CTT.GAC.AGA.CAC3' and a second primer comprises the sequence 5'ACA.AGG.TGC.TCA.CGC.ACA.TTG.ATC3'.

19. A kit according to claim 18 further comprising a probe for the detection of the amplified product of said virus nucleic acid.

20. A kit according to claim 18 or claim 19, further comprising an internal control nucleic acid capable of being amplified by using said first and second primer.

21. A kit according to anyone of the claims 18-20, further comprising a probe for the detection of amplified product of said internal control nucleic acid.

22. A method for reliably and routinely detecting and/or quantifying human cytomegalovirus in a clinical sample suspected of containing human cytomegalovirus nucleic acid comprising
- adding to said sample a known amount of internal control nucleic acid;
- isolating nucleic acid from said sample;
- optionally adding to said isolated nucleic acid another known amount of internal control nucleic acid;
- subjecting at least part of said nucleic acid to a nucleic acid amplification reaction using a first and a second primer;
- collecting any amplified products;
- performing a hybridisation reaction with at least part of said collected amplified product using a probe specific for amplified cytomegalovirus nucleic acid;
- performing a hybridisation reaction with at least part of said collected amplified product using a probe specific for said internal control nucleic acid;
- detecting and/or quantifying amplified internal control nucleic acid and any amplified cytomegalovirus nucleic acid;
wherein:
- said first primer comprises the sequence 5'CAC.TGG.CTC.AGA.CTT.GAC.AGA.CAC3' and said second primer comprises the sequence 5'ACA.AGG.TGC.TCA.CGC.ACA.TTG.ATC3';
- said internal control nucleic acid can be amplified by using said first and said second primer;
- said internal control nucleic acid comprises a sequence which allows the distinction of amplified products of said virus nucleic acid from amplified products of said internal control nucleic acid.

23. A method according to claim 22, wherein a high efficiency DNA isolation buffer, preferably buffer L7A comprising alpha casein, is used in the procedure for isolating nucleic acid from said clinical sample.

24. A method according to claim 22 or claim 23, wherein excess primers are removed after amplification, preferably through a Delta Y-A protocol.


SEQUENCE LISTING

(1) GENERAL INFORMATION:

(i) APPLICANT:
(A) NAME: Universiteit van Amsterdam
(B) STREET: Spui 21
(C) CITY: Amsterdam
(D) STATE: Noord-Holland
(E) COUNTRY: the Netherlands
(F) POSTAL CODE (ZIP): 1012 WX

(ii) TITLE OF INVENTION: A sensitive assay for the detection or
quantitation of human cytomegalovirus nucleic acid.

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98204421.6

(2) INFORMATION FOR SEQ ID NO: 1:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 1:

TGAAGGTCTT TGCCCAGTAC ATTCT
25

(2) INFORMATION FOR SEQ ID NO: 2:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 2:

AGAATGTACT GGGCAAAGAC CTTCA
25

(2) INFORMATION FOR SEQ ID NO: 3:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 3:

CCCTTTACAT CTTTCTGAAG TAGGG
25

(2) INFORMATION FOR SEQ ID NO: 4:

(i) SEQUENCE CHARACTERISTICS:
(A) LENGTH: 25 base pairs
(B). TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 4:

CCCTACTTCA GAAAGATGTA AAGGG
25

(2) INFORMATION FOR SEQ ID NO: 5:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 5:

CACTGGCTCA GACTTGACAG ACAC
24

(2) INFORMATION FOR SEQ ID NO: 6:

(i) SEQUENCE CHARACTERISTICS:
(A) LENGTH: 24 base pairs
(B) TYPE: nucleic acid

(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 6:

ACAAGGTGCT CACGCACATT GATC
24

(2) INFORMATION FOR SEQ ID NO: 7:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 7:

GATGAGGAGA GAGACAAGGT GC
22

(2) INFORMATION FOR SEQ ID NO: 8:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 8:

CTCAGACACT GGCTCAGACT TG
22

(2) INFORMATION FOR SEQ ID NO: 9:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 9:

TGACCTTATC AGTGTAATGA ACCGCCGCAT TGAGGAGATC TGCACCCTTT ACATCTTTCT
60

GAAGTAGGGG
70

(2) INFORMATION FOR SEQ ID NO: 10:

(i) SEQUENCE CHARACTERISTICS:
(A) LENGTH: 68 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 10:

CCCCCTACTT CAGAAAGATG TAAAGGGTGC AGATCTCCTC AATGCGGCGG TTCATTACAC
60

TGATAAGG
68

(2) INFORMATION FOR SEQ ID NO: 11:

(i) SEQUENCE CHARACTERISTICS:
(A) LENGTH: 7 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 11:

CCTGAGG
7

(2) INFORMATION FOR SEQ ID NO: 12:

(i) SEQUENCE CHARACTERISTICS:
(A) LENGTH: 7 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 12:

CCTGACC
7

(2) INFORMATION FOR SEQ ID NO: 13:

(i) SEQUENCE CHARACTERISTICS:
(A) LENGTH: 4 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 13:

GCGC
4

(2) INFORMATION FOR SEQ ID NO: 14:

(i) SEQUENCE CHARACTERISTICS:
(A) LENGTH: 4 base pairs
(B) TYPE: nucleic acid
(C) STRANDEDNESS: single
(D) TOPOLOGY: linear

(ii) MOLECULE TYPE: other nucleic acid

(iii) HYPOTHETICAL: NO

(xi) SEQUENCE DESCRIPTION: SEQ ID NO: 14:

CCGC
4

(2) INFORMATION FOR SEQ ID NO: 15:

(i) SEQUENCE CHARACTERISTICS:
(A) LENGTH: 597 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 15:

GATGAGGAGA GAGACAAGGT GCTCACGCAC ATTGATCACA TATTTATGGA TATCCTCACT
60

ACATGTGTGG AAACAATGTG TAATGAGTAC AAGGTCACTA GTGACGCTTG TATGATGACC
120

ATGTACGGGG GCATCTCTCT CTTAAGTGAG TTCTGTCGGG TGCTGTGCTG CTATGTCTTA
180

GAGGAGACTA GTGTGATGCT GGCCAAGCGG CCTCTGATAA CCAAGCCTGA CCTTATCAGT
240

GTAATGAACC GCCGCATTGA GGAGATCTGC ACCCTTTACA TCTTTCTGAA GTAGGGGGGG
300

GCCGATCCTC TGAGAGTCTG CTCTCCTAGT GTGGATGACC TACGGGCCAT CGCCGAGGAG
360

TCAGATGAGG AAGAGGCTAT TGTAGCCTAC ACTTTGGCCA CCGCTGGTGC CAGTTCCTCT
420

GATTCTCTGG TGTCACCTCC AGAGTCCCCT GTACCCGCGA CTATCCCTCT GTCCTCAGTG
480

ATTGTGGCTG AGAACAGTGA TCAGGAAGAA AGTGAGCAGA GTGATGAGGA ACAGGAGGAG
540

GGTGCTCAGG AGGAGCAGGA GGACACTGTG TCTGTCAAGT CTGAGCCAGT GTCTGAG
597